(19) Europäisches Patentamt
European Patent Office
Office européen des brevets

(11) Numéro de publication : **0 659 472 A2**

(12) **DEMANDE DE BREVET EUROPEEN**

(21) Numéro de dépôt : **94402968.5**

(22) Date de dépôt : **21.12.94**

(51) Int. Cl.[6] : **B01F 17/00,** C11D 3/12, A62C 2/00, A61K 7/50, A01N 25/16, A01N 25/30, E21B 33/138

(30) Priorité : **22.12.93 US 171796**

(43) Date de publication de la demande : **28.06.95 Bulletin 95/26**

(84) Etats contractants désignés : **AT BE CH DE DK ES FR GB GR IE IT LI LU NL PT SE**

(71) Demandeur : **RHONE-POULENC SPECIALTY CHEMICALS CO.**
**Prospect Plains Road,**
**CN 7500**
**Cranbury, NJ 08512-7500 (US)**

(72) Inventeur : **Dahanayake, Manilal**
**17 Reed Drive North**
**Princeton Junction, N.J. (US)**
Inventeur : **Zhu, Shawn**
**77 Winchester Drive**
**East Windsor, N.J. (US)**

(74) Mandataire : **Seugnet, Jean Louis et al**
**RHONE-POULENC CHIMIE,**
**Direction de la Propriété Industrielle,**
**25, Quai Paul Doumer**
**F-92408 Courbevoie Cédex (FR)**

(54) **Composition tensioactive caractérisée par une amélioration de la hauteur de la mousse.**

(57) Composition tensioactive moussable caractérisée par une augmentation de la hauteur de mousse, contenant comme tensioactif :

a) un tensioactif zwitterionique ou

b) un tensioactif non ionique à base d'alkylphénol éthoxylate ou

c) un mélange de deux ou plusieurs tensioactifs non ioniques, anioniques, cationiques et zwitterioniques en une quantité totale suffisante pour produire une mousse dans l'eau et

d) un polygalactomannane en une quantité suffisante pour augmenter la hauteur de mousse d'une solution aqueuse dudit tensioactif d'au moins 5 mm par rapport à un témoin du tensioactif sans polygalactomannane.

Lesdites compositions produisent pour une même teneur en tensioactif une augmentation de la hauteur de mousse ou pour une teneur moindre en tensioactif la même hauteur de mousse.

EP 0 659 472 A2

La présente invention concerne des compositions tensioactives caractérisées par une amélioration de la moussabilité permettant la mise en oeuvre de quantités moindres de tensioactif sans sacrifier la moussabilité.

Le pouvoir moussant des tensioactifs ou de leurs mélanges est une de leurs propriétés importantes qui trouve une utilité significative dans des domaines d'application aussi divers que les produits de soins corporels, les produits industriels et hospitaliers, les applications agricoles, la lutte contre les incendies et la consolidation des sols. On a déployé des efforts considérables de développement pour améliorer ou augmenter le pouvoir moussant des tensioactifs ou de leurs mélanges en vue de ces applications. Il est connu qu'il est difficile d'améliorer la hauteur de mousse d'une solution aqueuse de tensioactif ou d'un mélange de tensioactifs de plus de quelques millimètres en leur ajoutant un autre produit chimique. Généralement, les solutions de différents mélanges de tensioactifs ne présentent pas de synergie en matière de moussage ; il est difficile d'obtenir qu'un mélange de tensioactifs surpasse les performances individuelles de chacun des tensioactifs.

L'augmentation de la mousse est mesurée de trois manières, à savoir la hauteur de la mousse mesurée en millimètres après avoir laissé tomber la solution de tensioactif goutte à goutte dans un récipient à partir d'une hauteur déterminée (test de Ross-Miles), la densité de la mousse mesurée par le nombre de cellules de mousse par volume donné et la stabilité de la mousse mesurée également par le test de Ross-Miles et exprimée soit en durée (en secondes) nécessaire pour que la mousse diminue de la moitié de son volume initial ou en hauteur de mousse (en millimètres) au bout d'un temps donné. La présente invention est orientée plus particulièrement sur l'augmentation de la hauteur de mousse.

Il est connu que, en règle générale, la formation de mousse augmente au fur et à mesure que la concentration du tensioactif augmente jusqu'à ce que soit obtenue une concentration (qui est généralement supérieure à la concentration critique de micelles (CMC), c'est-à-dire la concentration à laquelle commence la formation de micelles) au-delà de laquelle la production de mousse reste, pour l'essentiel, constante à condition que la viscosité de la solution n'ait pas augmenté jusqu'à un point où la hauteur de mousse diminue. En règle générale, une viscosité importante de la solution se traduit par un effet négatif sur la production de mousse.

Il est bien connu que l'addition de polygalactomannanes, par exemple, de gomme Guar, aux tensioactifs améliore la stabilité de la mousse. Les quantités de gomme Guar que l'on enseigne d'ajouter pour augmenter la stabilité de la mousse ne sont pas connues pour affecter de manière significative ou pour augmenter la hauteur de la mousse.

Sachant qu'une viscosité importante limite la production de mousse, on peut augmenter la moussabilité en augmentant la teneur en tensioactif. Cependant, plus la quantité de tensioactif est élevée ou plus la stabilité de la mousse est importante, plus grande est la tendance des eaux usées à mousser, ce qui entraîne une utilisation accrue d'anti-mousse dans le traitement des eaux usées, que ce soit dans les systèmes industriels ou municipaux de traitement des eaux. Un produit moussable prenant en compte l'environnement aurait la possibilité d'assurer la hauteur de mousse voulue avec une stabilité suffisante pour l'application envisagée tout en utilisant, cependant, une quantité moindre de tensioactif de façon à ce qu'il soit moins nécessaire de procéder à un traitement anti-mousse extensif avant de traiter les eaux usées.

Les brevets EP-A 0 231 997 et EP-A 0 247 766 décrivent des formulations de shampooings contenant, entre autres, de la (des) gomme(s) Guar.

Or, on a trouvé de façon inattendue que les tensioactifs associés aux polysaccharides, tels que, par exemple, les polygalactomannanes, en quantités contrôlées inférieures à celles utilisées normalement pour assurer la stabilité de la mousse permettent d'augmenter la production de mousse, c'est-à-dire la hauteur de la mousse.

Selon la présente invention, on a trouvé que les compositions tensioactives caractérisées par une amélioration du pouvoir moussant ou de la hauteur de mousse peuvent être obtenues par combinaison d'un ou de plusieurs tensioactifs avec une quantité de polygalactomannane suffisante pour accroître le pouvoir moussant, c'est-à-dire la hauteur de mousse, d'au moins 5 mm, de préférence d'au moins 7,5 mm et plus particulièrement d'au moins 10 mm par rapport à la hauteur de mousse obtenue avec la même quantité de tensioactifs sans addition de polygalactomannane(s). Or, à la surprise générale, la mise en oeuvre de quantités de polygalactomannanes inférieures à celles qui sont enseignées pour augmenter la stabilité de la mousse, a pour effet inattendu d'augmenter la hauteur de mousse, ce qui permet d'utiliser moins de tensioactif pour obtenir la hauteur de mousse voulue.

La présente invention sera mieux comprise à la lecture de la description détaillée ci-après, référence sera faite au dessin annexé sur lequel :

La Figure 1 est un graphique représentant l'effet d'une quantité constante de gomme Guar doublement substituée sur la hauteur de mousse lorsque l'on fait varier les quantités d'un mélange de tensioactif anionique et de tensioactif zwitterionique par rapport à un témoin ne contenant pas de gomme Guar.

La Figure 2 est un graphique représentant l'effet que l'on obtient sur la hauteur de mousse d'un mélange d'un tensioactif anionique et d'un tensioactif zwitterionique lorsque l'on fait varier les quantités de gomme Guar doublement substituée.

La Figure 3 est un graphique représentant l'effet d'une quantité constante de gomme Guar doublement substituée sur la hauteur de mousse lorsque l'on fait varier les rapports entre le tensioactif anionique et le tensioactif cationique par rapport à un témoin ne contenant pas de gomme Guar.

La Figure 4 est un graphique représentant l'effet d'une quantité constante de gomme de guar hydroxypropylée sur la hauteur de mousse lorsque l'on fait varier les rapports entre le tensioactif zwitterionique et le tensioactif anionique par rapport à un témoin ne contenant pas de gomme Guar.

La Figure 5 est un graphique représentant l'effet d'une quantité constante d'un mélange 1 : 1 de gomme Guar carboxyméthylée et de gomme xanthane sur la hauteur de mousse lorsque l'on fait varier les quantités d'un mélange de tensioactifs par rapport à un témoin ne contenant pas de mélange gomme Guar/gomme xanthane.

La Figure 6 est un graphique représentant l'effet d'une quantité constante de gomme de guar carboxyméthylée sur la hauteur de mousse lorsque l'on fait varier les quantité d'un mélange de tensioactifs par rapport à un témoin ne contenant pas de gomme Guar.

Les compositions moussables selon la présente invention contiennent une composition tensioactive choisie dans le groupe comprenant les tensioactifs cationiques, anioniques non ioniques, zwitterioniques ainsi que leurs mélanges. La composition moussable peut contenir un tensioactif zwitterionique (lorsqu'il est utilisé seul) ou des mélanges d'un ou de plusieurs autres tensioactifs choisis parmi les autres types et leurs mélanges. Il tombe sous le sens que les tensioactifs sont choisis parmi des tensioactifs chimiquement compatibles utilisés dans la pratique de ce domaine.

Les tensioactifs cationiques sont des composés tensioactifs qui possèdent une charge positive sur la partie hydrophile de la molécule. Ces composés peuvent être représentés par les alkyl imidazolines, telle que l'oléyl imidazoline, les amines éthoxylées, telle que l'amine éthoxylée de suif, 5 - 15 EO (à 5 - 15 motifs oxyde d'éthylène), les dérivés quaternaires d'imidazoline, tel que le disuif imidazolinium méthyl sulfate, les dérivés quaternaires de benzyle, tel que le chlorure de benzyl trimonium, et les dérivés quaternaires de tétra alkyle, tel que le chlorure de cétyl trimonium. On a trouvé que certains dérivés quaternaires de tétra alkyle, lorsqu'ils sont mis en oeuvre seuls, ne donnent de hauteurs de mousse d'au moins 5 mm.

Le composé tensioactif anionique que l'on préfère le plus est un alkyl sulfate, un alkyl éther sulfate ou leurs mélanges. Les composés tensioactifs anioniques de ce type, utilisés communément, sont les alkyl sulfates en $C_{10}$-$C_{18}$ et les alkyl éther sulfates en $C_{10}$-$C_{18}$ contenant de 0 à 5, de préférence 3 moles ou plus d'oxyde d'éthylène. Lesdits tensioactifs sont généralement employés sous la forme de leurs sels de sodium, potassium, ammonium ou de mono, di ou triéthanolamine. Les exemples de ces tensioactifs sont le lauryl sulfate de sodium, le lauryl sulfate d'ammonium, les lauryl sulfates de mono, di et triéthanolammonium, le lauryl éther sulfate de sodium (2 EO), le lauryl éther sulfate de sodium (3 EO), le lauryl éther sulfate de potassium (2 EO) et le lauryl éther sulfate d'ammonium (3 EO).

D'autres composés tensioactifs anioniques appropriés sont les dialkyl succinates ayant la structure suivante :

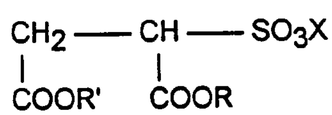

dans laquelle R et R' représentent les groupes alkyle à chaîne linéaire ou ramifiée, identiques ou différents, ayant de 5 à 14 atomes de carbone et R peut être égal à X, X étant un cation solubilisant choisi dans le groupe comprenant l'alcalin, l'ammonium, l'ammonium substitué et le magnésium.

Les dialkyl sulfosuccinates que l'on préfère particulièrement sont ceux dans lesquels R représente des groupes alkyle en $C_6$ et/ou en $C_8$.

D'autres composés tensioactifs anioniques qui conviennent également sont les α-oléfine sulfonates, les alkyl sarcosinates, les alkyl monoglycéride sulfates, les alkyl monoglycéride sulfonates, les alkyl benzène sulfonates, les monoalkyl éther sulfosuccinates, les alkyl éther carboxylates, les mono et diphosphate esters (alkyl, alkaryl et éthoxylate phosphate esters), y compris les phosphate esters présentant des rapport plus élevés entre les mono et les di acyl isothionates et acyl méthyl taurates.

Les tensioactifs non ioniques sont les tensioactifs qui ne possèdent pas de charge électrique et qui peuvent être représentés par les alkanolamides, les alkyl phénol éthoxylates, les éthoxylates d'acides gras, les éthoxylates d'alcool, les diesters de polyéthylèneglycol, les esters de glycérol, les uronates de galactoside et les alkylpoly glycosides. Les exemples de ces composés tensioactifs non ioniques comprennent le produit de la réaction entre un monolaurate de sorbitanne ou un monococoate de sorbitanne comportant de 20 à 80 moles d'oxyde d'éthylène et un alcool gras éthoxylé en $C_8$-$C_{16}$ comportant de 4 à 25 moles d'oxyde d'éthylène.

Les exemples de composés tensioactifs zwitterioniques, y compris aussi ceux que l'on appelle amphotères, comprennent les oxydes d'amine, tel que l'oxyde de lauramine, les bétaïnes, par exemple, les alkyl amido bétaïnes, telles que la cocamidopropyl bétaïne, l'alkyl diméthyl bétaïne, les sulfobétaïnes, (sultaïne, par exemple, l'alkyléther hydroxypropyl sultaïne, telle que la cocamidopropyl hydroxysultaïne) et les dérivés de l'imidazoline, par exemple, les coco, capryl, tallo, lauro ou stéarocamphocarboxylates de sodium ou disodium, tels que les acétates, diacétates, propionates, dipropionates et leurs contreparties acides, ainsi que leurs hydroxyalkyl sulfonates, iminocarboxylates, tels que les acétates, diacétates, propionates et dipropionates, et l'alkylglycine, par exemple, le dihydroxyéthyl glycinate de suif.

La quantité de composé tensioactif présente dans les compositions moussables selon l'invention est fonction de la forme de la composition finale. Par exemple, le produit moussable peut se trouver dans un liquide ou un gel exempts de propulseur, destinés à être versés, pompés ou distribués de toute autre manière à partir d'une bouteille ou d'un récipient similaire ou à partir d'un berlingot. Le produit moussable peut se présenter aussi sous la forme d'un liquide ou d'un gel contenant un propulseur liquéfiable gazeux, destiné à être utilisé à partir d'une bombe aérosol, telle une mousse, par exemple.

En règle générale, le tensioactif est présent en quantité suffisante pour assurer une production de mousse efficace dans son domaine d'application. Certains domaines d'application exigent une production de mousse importante, tels que les détergents pour petits lavages, les produits de nettoyage des moquettes, les produits de soins corporels, y compris les shampooings, les préparations pour le bain, les savons liquides, les mousses à raser, les savonnettes, les produits de soins pour les cheveux et la toilette, les mousses pour les applications de produits agricoles et de pesticides et les mousse de lutte contre le feu. En général, les mousses efficaces peuvent être obtenues en mettant en oeuvre environ de 20 à 0,01 % et, de préférence, environ de 5 à 0,2 % en poids de tensioactif dans la composition moussante finale.

Les quantités individuelles de composés tensioactifs cationiques, anioniques, non ioniques et zwitterioniques, seuls ou en mélange, se situent dans les limites de la quantité totale du composé tensioactif définies plus haut, à savoir entre 20 et 0,01 %, de préférence entre 5 et 0,2 % en poids par rapport à la composition finale.

Les compositions moussantes selon l'invention contiennent aussi un polygalactomannane polymère sous forme naturelle ou sous forme de dérivés ou de leurs mélanges. Les gommes de polygalactomannanes et leurs dérivés que l'on peut utiliser selon l'invention se trouvent dans la nature sous la forme de polysaccharides constitués principalement de motifs galactose et mannose. Habituellement, on trouve les polygalactomannanes dans l'endosperme des graines de légumineuses, tels que le guar, la caroube, le gleditschia, le flamboyant, le caféier du Kentucky et autres similaires. Le polygalactomannane que l'on préfère particulièrement pour sa mises en oeuvre dans le procédé selon la présente invention est la gomme Guar qui est le principal composant de la graine de la plante qui donne la gomme Guar, à savoir Cyamopsis tetragonalobus.

Le motif de base du galactomannane polymère dans la gomme Guar contient deux motifs mannose comportant une liaison glycosidique et un motif galactose relié à l'un des hydroxyles du motif mannose. En moyenne, chacun des motifs sucre possède trois sites hydroxyle disponibles, chacun d'eux pouvant réagir. Le degré de réaction ou de dérivatisation des groupes hydroxyle est appelé substitution molaire (S.M.) qui est le nombre de motifs (moles d'agent de dérivatisation) qui a réagi par motif sucre du polygalactomannane, ou degré de substitution (D.S.) qui est le nombre moyen de groupes hydroxy des motifs sucre que l'on a fait réagir avec l'agent de dérivatisation.

L'endosperme de Guar tel qu'il est utilisé dans la présente invention est appelé communément "séparé purifié" ou "séparé purifié deux fois" selon le degré de purification. Le "séparé purifié" est obtenu par séparation mécanique de l'endosperme de l'enveloppe et du germe de la graine de Guar sous une forme aussi pure et intacte que possible sans aucune autre étape de traitement. Ledit séparé purifié contient comme impuretés environ de 6 à 12 % d'humidité, de 2 à 7 % de protéines et de 2 à 7 % de résidu acide insoluble. Sa granulométrie est comprise entre environ 0,84 et 4,76 mm.

On peut faire réagir les particules de Guar avec divers agents de dérivatisation en milieu alcalin aqueux. On peut utiliser n'importe quel hydroxyde alcalin, mais la préférence va à l'hydroxyde de sodium. Le milieu réactionnel est l'eau, le procédé ne nécessitant aucun solvant organique.

Les dérivés des polygalactomannanes, tels que les dérivés à base d'hydroxyalkyl éther, d'alkyl éther, de carboxyalkyl éthers, d'aminoalkyl éther et d'alkyl éther d'ammonium quaternaire, sont des composés bien connus et il existe de nombreuses descriptions de la préparation de ces dérivés.

Les agents de dérivatisation sont les agents d'alkylation ou d'éthérification qui contiennent des groupes susceptibles de réagir avec les groupes hydroxyle des polygalactomannanes en vue de former des groupes éther, lesdits groupes réactifs étant des groupes époxyde, des atomes d'halogènes ou des groupes à insaturation éthylénique voisins. Les exemples de ces agents sont les agents d'alkylation, les agents d'hydroxyalkylation, les agents de carboxyalkylation, les agents d'aminoalkylation, les agents d'alkylation d'ammonium quaternaire, les agents d'amidoalkylation et autres similaires. Les agents d'alkylation comprennent le chlorure de méthyle, le bromure de méthyle, le chlorure d'éthyle, l'iodure d'éthyle et le chlorure d'isopropyle. Les agents d'hydroxyalkylation comprennent l'oxyde d'éthylène, l'oxyde-1,2 de propylène, l'oxyde-1,2 de butylène, l'oxyde-1,2 d'hexylène, la chlorohydrine d'éthylène, la chlorohydrine de propylène et l'épichlorohydrine. Les exemples d'agents de carboxyalkylation sont l'acide chloracétique, l'acide chloropropionique et l'acide acrylique. Les agents d'aminoalkylation comprennent le chlorure d'aminoéthyle, le bromure d'aminopropyle, le chlorure de N,N-diméthyl-aminopropyle et autres similaires. Les agents d'alkylation d'ammonium quaternaire sont des agents tels que le chlorure de 2,3-époxypropyl triméthylammonium, le chlorure de 3-chloro-2-hydroxypropyl triméthylammonium et autres similaires. Les agents contenant des groupes à insaturation éthylénique réagissant par l'intermédiaire de la réaction de Michael avec des groupes hydroxyle sont l'acrylamide, la méthacrylamide, l'acrylonitrile, le méthacrylonitrile, l'acide acrylique, l'acrylate de sodium et tout monomère polymérisable contenant un groupe polymérisable à insaturation éthylénique.

Les hydroxyalkyl éthers de polygalactomannanes peuvent être obtenus en faisant réagir les polygalactomannanes avec des oxydes d'alkylène dans des conditions basiques. Dans les brevets US-A 3 723 408 et 3 723 409 on fait réagir la farine de Guar avec des oxydes d'alkylène en présence d'eau et d'hydroxyde de sodium. Le produit de la réaction est neutralisé ensuite avec un acide, lavé avec un mélange d'alcool et d'eau, séché ensuite et broyé. Dans les brevets US-A 3 483 121, les polygalactomannanes et les oxydes d'alkylène réagissent en milieu basique avec de faibles quantités d'eau et des quantités plus importantes de solvants organiques miscibles ou immiscibles à l'eau.

Les carboxyalkyl éthers et les carboxyhydroxyalkyl éthers mixtes de polygalactomannanes sont décrits respectivement dans les brevets US-A 3 740 388 et 3 723 409. Ces agents de substitution (acide gras halogéné et oxyde d'alkylène) sont obtenus dans un mélange d'eau et d'alcool et lavés ensuite dans des mélanges d'eau et d'alcool.

D'autres dérivés de polygalactomannanes sont décrits dans des brevets tels que les brevets US-A 2 461 502 (cyanoéthyl éthers), US-A 4 094 795 (dialkylacrylamide éthers) et US-A 3 498 912 (alkyl éthers d'ammonium quaternaire). Dans les procédés décrits, les réactions se font dans des mélanges d'eau et de solvant organique et les produits de la réaction sont lavés avec des solvants ou avec des mélanges eau/solvant.

Pour éviter d'employer des liquides volatils inflammables et supprimer la nécessité de récupérer lesdits liquides organiques lorsque les réactions sont terminées, on a développé des procédés industriels qui utilisent uniquement l'eau comme milieu réactionnel. Dans ces procédés, on fait réagir l'endosperme du polygalactomannane avec l'agent de dérivatisation sous catalyse alcaline en utilisant suffisamment d'eau pour faire gonfler l'endosperme. Les produits obtenus sont lavés ensuite pour éliminer l'agent de dérivatisation qui n'a pas réagi, l'hydroxyde de sodium, le sel et les sous-produits. On peut utiliser du borax ou des sels d'aluminium pour réticuler les gels afin d'éviter la formation de gels extrêmement difficiles à manipuler et éviter d'avoir à laver le produit.

Les polygalactomannanes peuvent être classés parmi les produits non ioniques, y compris la gomme Guar naturelle et les hydroxy alkyl éthers, les produits anioniques, y compris les carboxyalkyl éthers, les produits cationiques, y compris les dérivés à base d'alkyl éther d'ammonium quaternaire, et les dérivés doubles, y compris les guars à base d'hydroxyalkyl éther/carboxyalkyl éther. Les gommes de polygalactomannane sont commercialisées par Rhône-Poulenc sous la marque JAGUAR.

Les guars se présentent généralement sous forme de poudre et sont hydratés dans une solution aqueuse de forte concentration avant d'être incorporés dans la solution tensioactive.

On a trouvé également de façon inattendue que l'utilisation de compositions à base de polygalactomannane fraîchement hydratée, à condition qu'elles soient utilisées rapidement après l'hydratation en vue d'obtenir de la mousse, c'est-à-dire moins d'un jour, donnent une hauteur de mousse supérieure à celle que l'on obtient avec quantité équivalente de gomme de polygalactomannane vieillie. Au-delà d'un jour de vieillissement, l'effet du guar sur la hauteur de mousse reste constante quel que soit l'âge de la solution de guar après hydratation. On a trouvé que l'on obtient une augmentation optimale de la hauteur de mousse si la gomme Guar est utilisée dans un délai inférieur à 3 heures et, de préférence, inférieur à 1 heure à compter de l'hydratation. Cet effet peut être particulièrement utile dans les applications industrielles et agricoles où l'on peut hydrater la gomme de polygalactomannane au moment de son utilisation. Les mousses de marquage agricoles, les mousses de traitement des semences ou les mousses d'application de pesticides, les mousses de consolidation des sols pourraient être plus efficaces si elles étaient préparées sur place et utilisées immédiatement. L'un de ces procédés d'application consiste à introduire le polygalactomannane dans de petits sacs séparés, le polygalactomannane étant séparé du ou des tensioactif(s) ou mélangé avec le ou les tensioactif(s), de façon à ce qu'ils soient dissous ou déchirés avant ou pendant le mélange.

La gomme et/ou le dérivé de polygalactomannane sont présents en quantité suffisante pour augmenter la hauteur de mousse au-delà de la hauteur que l'on peut obtenir en utilisant le ou les tensioactif(s) seul(s),

cette quantité étant insuffisante pour provoquer une diminution de la hauteur de mousse. La gomme de polygalactomannane est présente de préférence en une quantité comprise entre environ 0,005 % et environ 50 %, de préférence entre environ 0,05 et environ 15 % et plus particulièrement entre environ 0,02 % et environ 4 % en poids par rapport au poids du ou des composants tensioactifs (composant actif) de la mousse.

La composition moussante selon l'invention est un système aqueux et, au moment de la production de la mousse, elle contient généralement entre environ 80 % et environ 99,95 % d'eau. On peut obtenir des concentrés moussables en utilisant moins d'eau qu'il ne faut. Les concentrés moussables présentent le même rapport entre le polygalactomannane et le tensioactif que celui qui a été indiqué plus haut.

Les compositions moussables selon l'invention peuvent contenir en option divers composants particulièrement adaptés pour produire d'autres effets et utilisés couramment dans la fabrication de produits qui sont analogues aux compositions moussables et comprennent, sans y être limités, les colorants, parfums, sels, opacifiants, agents épaississants tels que les polymères hydrosolubles, les agents de mise en forme des cheveux et de la peau, les stabilisants, les agents de conservation et autres similaires.

Les compositions moussables selon la présente invention produisent une grande quantité de mousse avec moins de tensioactif, lesdites mousses étant suffisamment stables pour leur usage initial et pouvant être facilement brisées pour en faciliter l'élimination que ce soit dans l'environnement du consommateur ou dans celui de l'industrie. La mise en oeuvre de quantités moindres de tensioactif pour obtenir un niveau acceptable de mousse avec la possibilité, en plus, de briser facilement cette mousse permet de disposer de compositions écologiques qui sont plus faciles à éliminer au cours du traitement des eaux usées. Ces propriétés permettent d'adapter lesdites compositions à des utilisations dans des produits qui vont des cosmétiques aux applications industrielles et qui peuvent être mis en oeuvre partout où les compositions moussables ont trouvé une application.

Ces produits sont particulièrement utiles dans les shampooings, y compris les shampooings pour bébés, les bains moussants, y compris les bains à bulles, les savonnettes, les gels de bain, les gels de mise en forme des cheveux, les lotions, les crèmes et lotions pour la peau, les crèmes et lotions de démaquillage, les détergents liquides, les détergents pour la vaisselle et autres produits de lavage et cosmétiques, les mousses de marquage agricoles, les mousses de traitement des semences, les mousses d'application de pesticides, les mousses de consolidation des sols et les mousses de lutte contre le feu.

Les exemples qui suivent illustrent pleinement la présente invention. Le pourcentage des composants des produits préparés dans les exemples est donné par rapport au poids total de la solution les pourcentages donnés dans les revendications sont des pourcentages en poids. Les guars utilisés dans les exemples suivants se présentent sous la forme de poudre et sont hydratés dans une solution aqueuse de concentration relativement élevée avant d'être incorporés dans la solution tensioactive.

EXEMPLE 1

Pour démontrer l'efficacité de l'invention, on combine divers tensioactif(s) avec diverses gommes Guar et on procède à une comparaison par rapport à des formulations témoins ne contenant pas de gommes Guar. Les quantités de tensioactifs et de gomme Guar sont maintenues constantes.

Les tensioactifs et polysaccharides qui sont étudiés et dont on rend compte dans les exemples sont présentés dans les TABLEAUX I et II ci-après :

TABLEAU I

| Classe du tensioactif | Sous-classe du tensioactif | Marque du tensioactif | Nom chimique du tensioactif | Nom abrégé du tensioactif |
|---|---|---|---|---|
| Zwitterioniques | Oxyde d'amine | RHADAMOX LO | Oxyde de lauramine | LO |
| | Amphoglacinate | MIRANOL C2M | Cocoamphodiacétate de sodium | C2M |
| | Amphoglacinate | DV4209 ou CMA | de sodium | CMA |
| | Bétaïne | MIRATAINE CB | Cocoamidopropyl bétaïne | CB |
| | Bétaïne | MIRATAINE CBC | Cocoamidopropyl bétaïne | CBC |
| | Bétaïne | MIRATAINE H2C-HA | Lauryiminodipropionate de sodium | H2CHA |
| | Sultaïne | MIRATAINE CBS | Cocaamidopropyl hydroxysultaïne | CBS |
| Cationiques | (composé quaternisé) | RHODAQUAT M242C/29 | Chlorure de dodécyltriméthylammonium | DTAC |
| Anioniques | Alkylsulfate | RHODAPONSB8208/s | Docécyl sulfate de sodium | SB |
| | Sulfonate | RHODACAL A246L | Oléfine sulfonate de sodium | A246 |
| | Sulfonate | RHODACAL DS-4 | Dodécyl benzène sulfonate de sodium | DS-4 |
| | Alkyléther sulfate | RHODAPEX ES | Laureth (3) sulfate de sodium | ES |
| | Alkyléther sulfate | RHODAPEX ES2 | Laureth (2) sulfate de sodium | ES2 |
| | Alkyléther sulfate | RHODAPEX ESY | Laureth (1) sulfate de sodium | ESY |
| | Alkylphosphate ester | DV3999 | Monoryl phosphate ester supérieur | DV3999 |
| | Taurate | GEROPON T-77 | N-méthyl-N-oléoytaurate de sodium | T-77 |
| Non ioniques | Alkylanolamide | ALKAMIDE DC 212/S | Cacamide DEA (1 : 1) | DC212 |
| | Ethoxylate | IGEPAL CO-630 | Nonylphénol (9 EO) | CO630 |
| | Alcool éthoxylate | RHADASURF L-4 | Lauryl alcool éthoxylate | C12E4 |
| Biodégradable | Galactoside uronate | | Décyl-D galactoside uronate de sodium | SDDGU |
| | Glycoside | GLUCOPON 625 CS | Alkyl polyglycoside | APG625 |

TABLEAU II

| Classe du polymère | Sous-classe du polymère | Marque du polymère | Nom chimique du polymère | Nom abrégé du polymère |
|---|---|---|---|---|
| Non ioniques | Gomme Guar naturelle | JAGUAR 2100 | Gomme Guar | 2100 |
| | Gomme Guar modifiée | JAGUAR 2513 | Gomme Guar | 2513 |
| | Gomme Guar modifiée | JAGUAR 2638 | Gomme Guar | 2638 |
| | Guar hydroxypropylée | JAGUAR HP-8 | 2-hydroxypropyl éther | HP8 |
| | Guar hydroxypropylée | JAGUAR HP-120 | 2-hydroxypropyl éther | HP120 |
| Anioniques | Guar carboxyméthylée | JAGUAR CB-9 | Carboxyméthyl éther de sodium | CB-9 |
| Cationiques | Dérivé cationique de guar hydroxypropylée | JAGUAR C-162 | Chlorure de 2-hydroxypropyl-2-hydroxy-3-(triméthylammonio)propyl éther | C162 |
| Dérivé doublement substitué | Guar hydroxypropylée-carboxyméthylée | JAGUAR 8600 | | 8600 |

On évalue le pouvoir moussant du produit en effectuant le test de hauteur de mousse de Ross-Miles tel qu'il est exposé dans la norme ASTM D1173 et on compare avec un système témoin sans ajout de polymère, c'est-à-dire de polygalactomannane. Le test consiste à laisser tomber goutte à goutte depuis une hauteur déterminée une quantité déterminée de solution de tensioactif dans un récipient de dimensions normalisées, à mettre en oeuvre un volume constant de solution, à savoir 250 millilitres, à une température constante et à enregistrer la hauteur initiale de mousse au-dessus des 250 millilitres (Hauteur de Mousse initiale - HMi), la hauteur totale de mousse au-dessus du niveau du liquide à zéro minute (Hauteur de Mousse flash) et la hauteur de mousse au-dessus des 250 millilitres au bout de 5 minutes (Hauteur de Mousse après 5 minutes - HM5). On évalue la mousse et on obtient les résultats suivants qui sont rassemblés dans le tableau III ci-après :

## TABLEAU III

**HAUTEUR DE MOUSSE SELON LE TEST DE ROSS-MILES DE SOLUTIONS DE TENSIOACTIF SEUL ACTIF A 1,0 % ET DE LEURS MELANGES BINAIRES (0,5 % : 0,5 %) AVEC UN POLYMERE (SOLUTION VIEILLIE A 0,02 %) ET SANS POLYMERE A TEMPERATURE AMBIANTE**

Polymère

| Tensioactif | Sans polymère | | | Jaguar 2100 | | | Jaguar HP-120 | | |
|---|---|---|---|---|---|---|---|---|---|
| | HMi | HMf | HM5 | HMi | HMf | HM5 | HMi | HMf | HM5 |
| C2M | 168 | 187 | 168 | 172 | 199 | 172 | 168 | 193 | 168 |
| CB | 170 | | 170 | 173 | 195 | 173 | 174 | 197 | 174 |
| LO | 170 | 185 | 30 | 184 | 205 | 184 | 182 | 201 | 184 |
| SE | 172 | 199 | 172 | | | | | | |
| ES | 170 | 189 | 170 | 173 | 199 | 173 | 170 | 198 | 170 |
| C2M/A246 | 176 | 199 | 176 | 178 | 208 | 178 | 172 | 199 | 172 |
| | | | | | | | | | |
| C2M/SB | 177 | 209 | 177 | 193 | 229 | 193 | 191* | 229 | 191 |
| C2M/ES | 174 | 198 | 174 | 176 | 211 | 176 | 175 | 206 | 175 |
| C2M/A246 | 179 | 205 | 179 | 186 | 220 | 186 | 182 | 215 | 182 |
| | | | | | | | | | |
| CB/SB | 172 | | 172 | 191 | 231 | 191 | 193* | 233 | 193 |
| CB/ES | 173 | 197 | 173 | 180 | 211 | 180 | 176 | 205 | 176 |
| | | | | | | | | | |
| LO/SB | 160 | 200 | 160 | 191 | 246 | 191 | 191 | 241 | 191 |
| LO/ES | 171 | 206 | 171 | 194 | 234 | 194 | 190 | 230 | 190 |
| LO/A246 | 170 | 201 | 170 | 192 | 231 | 192 | 189 | 227 | 189 |

## TABLEAU III (suite)

Polymère

| Tensioactif | Jaguar 8600 | | | Jaguar CB-9 | | | Jaguar C-162 | | |
|---|---|---|---|---|---|---|---|---|---|
| | HMi | HMf | HM5 | HMi | HMf | HM5 | HMi | HMf | HM5 |
| C2M | 171 | 198 | 171 | 166 | 192 | 166 | 166 | 190 | 166 |
| CB | 174 | 198 | 174 | 168 | 188 | 168 | 171 | | 171 |
| LO | 186 | 210 | 186 | 185 | 211 | 185 | 175 | 202 | 175 |
| SB | 176 | 205 | 176 | 175 | 205 | 175 | | | |
| ES | 172 | 198 | 172 | 174 | 202 | 174 | 166 | 192 | 166 |
| C2M/A246 | 177 | 206 | 177 | 177 | 210 | 177 | 173 | 200 | 173 |
| | | | | | | | | | |
| C2M/SB | 190 | 227 | 190 | 187 | 225 | 187 | | | |
| C2M/ES | 175 | 206 | 175 | 180 | 211 | 180 | 183 | 212 | 183 |
| C2M/A246 | 183 | 217 | 183 | 188 | 224 | 188 | | | |
| | | | | | | | | | |
| CB/SB | 190 | 230 | 190 | 189 | 229 | 189 | | | |
| CB/ES | 176 | 199 | 176 | 180 | 213 | 180 | 176 | 215 | 180 |
| | | | | | | | | | |
| LO/SB | 185 | 235 | 185 | 188 | 240 | 188 | 185 | 229 | 185 |
| LO/ES | 184 | 224 | 184 | 188 | 228 | 188 | 184 | 225 | 184 |
| LO/A246 | 184 | 221 | 184 | 185 | 224 | 185 | 180 | 221 | 180 |

* Jaguar HP-8

Nota : HMi = Hauteur de Mousse initiale, HMf = Hauteur de Mousse flash, HM5 = Hauteur de mousse après 5 minutes. L'unité de hauteur de mousse est exprimée en mm.

Effet de l'ajout de polymère (solution vieillie à 0,02 %) sur la hauteur de mousse de systèmes à base d'un seul tensioactif (actif à 1 %)

Les hauteurs de mousse flash et les hauteurs de mousse initiales de solutions de tensioactif à base d'oxyde d'amine (RHODAMOX LO) peuvent être augmentées respectivement de 16 à 27 mm et de 12 à 17 mm lorsque l'on y ajoute un polymère, tels que le JAGUAR 1200, le JAGUAR HP-120, le JAGUAR 8600, le JAGUAR CB-9 et le JAGUAR C-162 bien que le JAGUAR C-162 n'augmente la hauteur de mousse initiale que d'environ 5 mm.

Les hauteurs de mousse flash et les hauteurs de mousse initiales de solutions de tensioactif à base de nonylphénoxypoly(éthylèneoxy)éthanol (IGEPAL CO-630) peuvent être augmentées respectivement de 28 mm et de 22 mm lorsque l'on y ajoute 0,02 % de polymère, tel que le JAGUAR HP-120 et respectivement d'environ 45 mm et environ 26 mm avec 0,1 % de JAGUAR 8600.

On obtient une amélioration moins nette de la hauteur de mousse flash lorsque l'on met en oeuvre des tensioactifs zwitterioniques, tels que les amphoglacinates (par exemple, le MIRANOL C2M), les tensioactifs anioniques, tels que les alkyl lauryl sulfates (par exemple, le RHODAPON SB 8208/s et les sulfonates (par exemple, le RHODACAL A246/L). Le JAGUAR 2100 et le JAGUAR 8600 augmentent la hauteur de mousse flash du C2M de 12 mm et le JAGUAR HP-120, le JAGUAR CB-9 et le JAGUAR C-162 de quelques millimètres. Les JAGUAR 2100, JAGUAR HP-120, JAGUAR 8600 et le JAGUAR CB-9 augmentent la hauteur de mousse flash du RHODAPEX ES de l'ordre de 10 mm alors que le JAGUAR C-162 ne l'augmente que de quelques millimètres seulement. La hauteur de mousse flash des solutions de RHODAPON SB 8208/s n'est augmentée que d'environ 5 mm par le JAGUAR 8600 et le JAGUAR CB-9. Les JAGUAR 2100, JAGUAR 8500 et le JAGUAR CB-9 n'augmentent la hauteur de mousse flash du RHODACAL A246/L que de quelque 8 mm alors que le JAGUAR C-162 et le JAGUAR HP-120 n'affectent pas la hauteur de mousse flash des solutions de ce tensioactif.

On obtient une faible augmentation (quelques millimètres) ou bien aucune augmentation de la hauteur de mousse initiale dans les tensioactifs zwitterioniques, tels que les amphoglacinates (par exemple, le MIRANOL C2M), les tensioactifs anioniques, tel que l'alkyl lauryl sulfate (par exemple, le RHODAPON SB8208/s) et les sulfonates (par exemple, le RHODACAL A246/L).

Effet de l'ajout de polymère (solution vieillie à 0.02 %) sur la hauteur de mousse de systèmes tensioactifs binaires (1% de substance active)

Les hauteurs de mousse flash et les hauteurs de mousse initiales des solutions tensioactives à base d'oxyde d'amine (RHODAMOX LO) et de dodécylsulfate de sodium (RHODAPON SB8208/s) peuvent être augmentées respectivement de 30 à 46 mm et de 25 à 31 mm lorsque l'on ajoute 0,02 % de polymère, tels que le JAGUAR 12OO, le JAGUAR HP-120, le JAGUAR 8600 et le JAGUAR C-162.

Les hauteurs de mousse flash et les hauteurs de mousse initiales des solutions tensioactives à base d'oxyde d'amine (RHODAMOX LO) et de laureth (3) sulfate de sodium (RHODAPEX ES) peuvent être augmentées respectivement de 15 à 28 mm et de 13 à 22 mm lorsque l'on ajoute 0,02 % de polymère, tels que le JAGUAR 2100, le JAGUAR 8600, le JAGUAR HP-120, le JAGUAR CB-9 et le JAGUAR C-162.

Les hauteurs de mousse flash et les hauteurs de mousse initiales de solutions tensioactives à base d'oxyde d'amine (RHODAMOX LO) et d'oléfine sulfonate de sodium (RHODACAL A246L) peuvent être augmentées respectivement de 20 à 30 mm et de 15 à 22 mm lorsque l'on ajoute 0,02 % de polymère, tels que le JAGUAR 2100, le JAGUAR 8600, le JAGUAR HP-120, le JAGUAR CB-162, ce dernier augmentant la hauteur de mousse initiale de l'ordre de 6 à 10 mm.

Les hauteurs de mousse flash et les hauteurs de mousse initiales des solutions tensioactives à base de nonylphénoxypoly(éthylèneoxy)éthanol (IGEPAL CO-630) peuvent être augmentées respectivement de 28 et 22 mm lorsque l'on ajoute 0,02 % de polymère, tel que le JAGUAR HP-120, et respectivement d'environ 45 mm et 26 mm avec 0,1 % de JAGUAR 8600.

On a trouvé que les hauteurs de mousse flash et les hauteurs de mousse initiales sont augmentées (respectivement de 18 mm et d'environ de 10 à 16 mm) lorsque l'on utilise des tensioactifs zwitterioniques, tels que les amphoglacinates (par exemple, le MIRANOL C2M) en combinaison avec des tensioactifs anioniques, tels que les alkyl sulfates (par exemple, le dodécyl sulfate de sodium RHODAPON SB 8208/s) avec 0,02 % de polymère, par exemple, du JAGUAR 2100, du JAGUAR 2513, du JAGUAR 8600, du JAGUAR CB-9 et du JAGUAR HP-8.

Par rapport au témoin, on trouve des hauteurs de mousse flash améliorées de l'ordre de 13 mm avec le JAGUAR 2100, le JAGUAR CB-9 et le JAGUAR C-162 et de l'ordre de 7 mm avec le JAGUAR HP-120 et le JAGUAR 8600; on trouve des hauteurs de mousse initiales améliorées de l'ordre de 6 mm à 9 mm avec le JAGUAR CB-9 et le JAGUAR C-162 et une légère amélioration ou aucune amélioration avec le JAGUAR 2100,

le JAGUAR-120 et le JAGUAR 8600 lorsque l'on met en oeuvre des tensioactifs zwitterioniques, tels que les amphoglacinates (par exemple, le MIRANOL C2M) en combinaison avec des tensioactifs anioniques à base d'alkyléther sulfate, tel que le laureth (3) sulfate de sodium RHODAPEX ES.

Par rapport au témoin, on trouve des hauteurs de mousse flash améliorées de l'ordre de 10 à 20 mm avec le JAGUAR 2100, le JAGUAR CB-9 et le JAGUAR C-120; les hauteurs de mousse initiales sont augmentées de l'ordre de 7 mm avec le JAGUAR 2100 et le JAGUAR CB-9 et de quelques millimètres avec le JAGUAR HP-120 et le JAGUAR 8600 lorsque l'on utilise des tensioactifs zwitterioniques, tels que les amphoglacinates (par exemple, le MIRANOL C2M) en combinaison avec des tensioactifs à base de sulfonate, tel que l'oféfine sulfonate de sodium RHODACAL A246L.

Par rapport au témoin, on trouve des hauteurs de mousse flash améliorées de l'ordre de 15 mm avec le JAGUAR 2100, le JAGUAR CP-9 et le JAGUAR C-162 et de quelques millimètres avec le JAGUAR 8600 et le JAGUAR HP-120 ; les hauteurs de mousse initiales sont augmentées de l'ordre de 17 à 21 mm avec le JAGUAR 2100, le JAGUAR HP-8, le JAGUAR 8600 et le JAGUAR CB-9 lorsque l'on met en oeuvre des tensioactifs zwitterioniques, telles que les bétaïnes (par exemple, la cocoamidopropyl bétaïne MIRATAINE CB) en combinaison avec des tensioactifs, tels que les alkyl sulfates (par exemple, le dodécyl sulfate de sodium RHODAPON SB 8208/s).

On trouve des hauteurs de mousse initiales améliorées de l'ordre de 7 mm avec le JAGUAR 2100, le JAGUAR HP-8, le JAGUAR CB-9 et le JAGUAR C-162 et une légère amélioration ou aucune amélioration avec le JAGUAR HP-120 et le JAGUAR 8600 lorsque l'on met en oeuvre des tensioactifs zwitterioniques, telles que les bétaïnes (par exemple, la cocoamidopropyl bétaïne MIRATAINE CB) en combinaison avec des tensioactifs anioniques à base d'alkyléther sulfate, tel que le laureth (3) sulfate de sodium RHODAPEX ES.

EXEMPLE 2

On étudie d'autres tensioactifs selon le procédé de l'EXEMPLE 1 et on obtient les résultats suivants rassemblés dans le tableau IV ci-après :

TABLEAU IV

| % en poids | TA1 | en poids | TA2 | % en poids | Guar | HMi | HMf | HM5 |
|---|---|---|---|---|---|---|---|---|
| 0,32 | CB | + 0,17 | ESY | + 0,013 | CB-9 | 186 | | 186 |
| 0,32 | CB | + 0,17 | ES2 | + 0,013 | CB-9 | 182 | | 182 |
| 0,32 | CB | + 0,17 | DS-4 | + 0,013 | CB-9 | 182 | | 182 |
| 0,32 | CB | + 0,17 | A246 | + 0,013 | CB-9 | 186 | | 186 |
| 0,5 | CMA | | | | | 161 | | 161 |
| 0,25 | CMA | + 0,25 | SB | + 0,013 | CB-9 | 186 | | 186 |
| 0,05 | C2M | + 0,05 | SDDGU | + 0,02 | CB-9 | 160 | | 160 |
| 0,05 | C2N | + 0,05 | SDDGU | | | 156 | | 156 |
| 0,1 | C2M | | | | | 151 | | 151 |
| 0,1 | SDDGU | | | | | 146 | | 146 |
| 0,25 | CMA | + 0,25 | SB | + 0,02 | HP8 | 188 | | 188 |
| 0,5 | CBC | + 0,5 | SB | | | 175 | | 175 |
| 1 | CBC | | | | | 170 | | 170 |
| 0,5 | CBC | + 0,5 | SB | + 0,013 | CB-9 | 188 | | 188 |
| 0,75 | CMA | | | | | 163 | 178 | 163 |
| 0,75 | SB | | | | | 171 | 193 | 171 |
| 0,375 | CMA | + 0,375 | SB | | | 171 | 195 | 171 |
| 0,375 | CMA | + 0,375 | SB | + 0,008 | R-23 | 175 | 209 | 175 |
| 0,5 | CMA | + 0,5 | SB | + 0,011 | 8600 | 184 | 220 | 184 |
| 1 | CO630 | | | | | 145 | 157 | 137 |
| 0,5 | LO | + 0,5 | CO630 | | | 173 | 191 | 30 |
| 0,5 | LO | + 0,5 | CO630 | + 0,01 | 8600 | 183 | 205 | 183 |
| 1 | H2CHA | | | | | 166 | 182 | 166 |
| 1 | T77 | | | | | 156 | 176 | 155 |
| 0,5 | H2CHA | + 0,5 | T77 | | | 161 | 179 | 161 |
| 0,5 | H2CHA | + 0,5 | T77 | + 0,01 | 8600 | 169 | 189 | 169 |
| 1 | L22 | | | | | 179 | 205 | 179 |
| 1 | DC212 | | | | | 151 | 178 | 151 |
| 0,5 | L22 | + 0,5 | DC212 | | | 172 | 202 | 172 |
| 0,5 | L22 | + 0,5 | DC212 | + 0,01 | 8600 | 182 | 210 | 182 |
| 0,5 | CBC | + 0,5 | ES | + 0,01 | CB-9 | 180 | 211 | 180 |
| 0,45 | CBC | + 0,45 | SB | + 0,02 | CB-9 | 183 | 221 | 183 |
| 0,62 | C2M | + 0,48 | SB | + 0,02 | Méth | 170 | 204 | 170 |

EXEMPLE 3

On étudie les hauteurs de mousse que l'on obtient en mettant en oeuvre un système binaire 1 : 1 constitué d'un tensioactif anionique à base d'alkyl sulfate (par exemple, de dodécyl sulfate de sodium RHODAPON SB8208/s) en combinaison avec un tensioactif cationique à base d'oxyde d'amine (Oxyde de lauramine RHODAMOX LO) avec une gomme Guar doublement substituée (JAGUAR 8600). Les données qui figurent dans le TABLEAU V représentent les résultats que l'on obtient lorsque l'on fait varier la quantité de tensioactif tout en gardant constante à 0,01 % la quantité de gomme Guar de moins de 7 heures (données représentées sur la Figure 1), que l'on fait varier la quantité de gomme Guar de moins de 7 heures tout en gardant constante à 0,5 % en poids la quantité de mélange de tensioactifs (données représentées sur la Figure 2) et que l'on fait varier le rapport entre les tensioactifs, un $\alpha$ de zéro signifiant 0 % de RHODAPON SM8208/s et 100 % de RHODAMOX LO et un $\alpha$ de un signifiant 0 % de RHODAMOX LO et 100 % de RHODAPON SB8208/s avec des décimales d'égalisation des rapports entre les deux, la gomme Guar étant maintenue constante à 0,01 % et ayant moins de 5 heures (données représentées sur la Figure 3).

## TABLEAU V

Comparaison des hauteurs de mousse lorsque l'on met en oeuvre une quantité constante d'une gomme Guar doublement substituée et que l'on fait varier le rapport entre le tensioactif anionique SB 8208/s et le tensioactif cationique LO

Hauteur de mousse du système RHODAPON SB 8208/RHODAMOX LO/JAGUAR 8600

1 % en poids de tensioactif actif, 0,01 % de guar

| % en poids de TA | α | % en poids de guar | HMi | HMf | HM5 | pH |
|---|---|---|---|---|---|---|
| 1 | 1 | 0,01 | 190 | 223 | 190 | 8,9* |
| 1 | 0,5 | 0,01 | 202 | 252 | 202+6 | 9,25 |
| 1 | 0 | 0,01 | 187 | 211 | 170 | 6,9 |
| 1 | 1 | | 170 | | | 8,9** |
| 1 | 0,5 | | 160 | 200 | 160 | 9,25 |
| 1 | 0 | | 170 | 185 | 30 | 7,1 |
| 0,5 | 1 | 0,01 | | | | *** |
| 0,5 | 0,5 | 0,01 | 200 | 250 | 202 | |
| 0,5 | 0 | 0,01 | | | | |
| 0,5 | 0,5 | 0,005 | 195 | 245 | 195 | |
| 0,5 | 1 | | 170 | | | |
| 0,5 | 0,5 | 0 | 162 | 202 | 162 | |
| 0,5 | 0 | | | | | |
| 0,25 | 1 | 0,01 | | | | |
| 0,25 | 0,5 | 0,01 | 192 | 242 | 192 | |
| 0,25 | 0 | 0,01 | | | | |
| 0,25 | 1 | | | | | |
| 0,25 | 0,5 | | 165 | 207 | 165 | |
| 0,25 | 0 | | | | | |
| 0,125 | 1 | 0,01 | | | | |
| 0,125 | 0,5 | 0,01 | 183 | 228 | 183 | |
| 0,125 | 0 | 0,01 | | | | |
| 0,125 | 1 | | | | | |
| 0,125 | 0,5 | | 166 | 204 | 166 | |
| 0,125 | 0 | | | | | |

* L'ensemble de la mousse présente son maximum au centre

** Mousse très dense

*** 197 mm encore après 16 heures

EXEMPLE 4

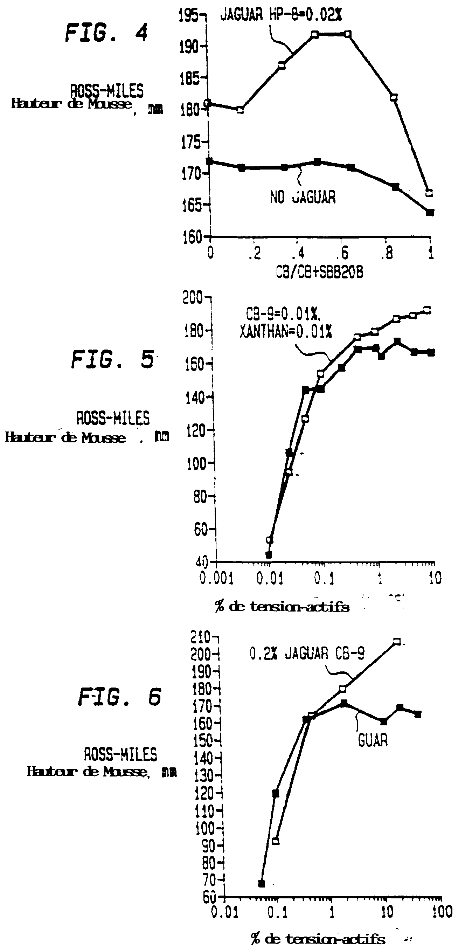

On procède comme dans l'exemple 1 en faisant varier les rapports entre un tensioactif zwitterionique à base de cocoamidopropyl bétaïne (MIRATAINE CB) et un tensioactif anionique à base de dodécylsulfate de sodium (RHODAPON SB8208/s) et en mettant en oeuvre 0,02 % d'un guar hydroxypropylé non ionique (JAGUAR HP68) à pH 6 et à 25°C ; on compare par rapport à un témoin ne contenant pas de guar. La teneur totale en tensioactif actif est de 0,5 % en poids. Les résultats obtenus sont reportés sur la FIGURE 4.

EXEMPLE 5

On obtient des hauteurs de mousse en mettant en oeuvre des mélanges tertiaires de tensioactifs, à savoir 0,5 % de lauryl alcool éthoxylate (RHODASURF L-4), 0,29 % de cocoamphodiacétate de sodium (MIRANOL C2M) et 0,21 % de dodécylsulfate de sodium (RHODAPON SB8208/s) plus 0,02 % de guar à base de carboxyméthyl de sodium (JAGUAR CB-9) et on compare par rapport à un témoin. Les hauteurs de mousse initiales et au bout de 5 minutes sont de 179 mm, les hauteurs flash de 199 mm.

EXEMPLE 6

On obtient des hauteurs de mousse en mettant en oeuvre 10 % d'un mélange de tensioactifs qui est un mélange ternaire de tensioactifs, à savoir de cocoamphodiacétate, de lauryl sulfate de sodium et de laureth sulfate de sodium (47 % de matières actives) plus 0,02 % de sodium carboxyl guar (JAGUAR CB-9). La hauteur de mousse flash est augmentée de 16 mm et la hauteur de mousse initiale de 9 mm par rapport à un témoin.

EXEMPLE 7

Les hauteurs de mousse sont obtenues en mettant en oeuvre de 10 à 0,25 % d'un mélange de tensioactifs qui est un mélange ternaire de tensioactifs, à savoir de cocoamide - diéthanolamide 1 / 1, de laureth sulfate de sodium et de cocamidopropyl bétaïne (47 % de matières actives) plus 0,01 % de sodium carboxyméthyl guar (JAGUAR CB-9) et 0,01 % de gomme xanthane. La hauteur de mousse flash est augmentée de 30 à 35 mm, la hauteur de mousse initiale de 10 à 25 mm par rapport à un témoin. Ces données sont reportées sur la FIGURE 5.

Lorsque l'on met en oeuvre une solution à 4,5 %, la hauteur de mousse initiale est augmentée de l'ordre de 14 à 20 mm et la hauteur de mousse flash de l'ordre de 16 à 19 mm avec 0,02 % de JAGUAR C-162 ou de JAGUAR 8600.

EXEMPLE 8

On obtient des hauteurs de mousse en mettant en oeuvre de 20 à 1,0 % d'un mélange de tensioactifs, à savoir 45 parties de lauryl sulfate de sodium, 8 parties de monobutyle d'éthylèneglycol, 8 parties de laureth (3) sulfosuccinate de sodium, 3 parties d'alcool en $C_{12}$-$C_{16}$, 5 parties de cocamide MEA, 25 parties de sel d'ammonium d'éthoxylate en $C_8$-$C_{10}$ sulfaté comme matières actives plus 0,02 % de sodium carboxyméthyl guar (JAGUAR CB-9). La hauteur de mousse flash est augmentée de 48 à 16 mm alors que la hauteur de mousse initiale est augmentée de 38 à 8 mm par rapport à un témoin. Ces données sont reportées sur la FIGURE 6

On peut procéder à diverses modifications dans les détails des différents modes de réalisation des compositions et procédés selon la présente invention, toutes ces modifications étant situées dans le domaine de l'invention défini par les revendications ci-après.

## Revendications

1. Composition tensioactive moussable caractérisée par une augmentation de la hauteur de mousse, contenant :
   a) un tensioactif zwitterionique comme seul tensioactif, en une quantité suffisante pour produire une mousse dans l'eau et
   b) un polygalactomannane, y compris des mélanges, en une quantité suffisante pour augmenter la hauteur de mousse initiale d'une solution aqueuse dudit tensioactif zwitterionique d'au moins 5 mm par rapport à un témoin sans polygalactomannane avec mise en oeuvre du test de mousse selon Ross-Miles.

2. Composition tensioactive moussable caractérisée par une augmentation de la hauteur de mousse, contenant :
   a) un tensioactif non ionique à base d'alkylphénol éthoxylate comme seul tensioactif, en une quantité suffisante pour produire une mousse dans l'eau et
   b) un polygalactomannane, y compris des mélanges, en une quantité suffisante pour augmenter la hauteur de mousse initiale d'une solution dudit tensioactif non ionique d'au moins 5 mm par rapport à un témoin de tensioactif sans polygalactomannane avec mise en oeuvre du test de mousse selon Ross-Miles.

3. Composition tensioactive caractérisée par une augmentation de la hauteur de mousse, contenant :
a) un mélange de deux ou plusieurs tensioactifs non ioniques, anioniques, cationiques et zwitterioniques en une quantité totale suffisante pour produire une mousse dans l'eau et
b) un polygalactomannane, y compris des mélanges, en une quantité suffisante pour augmenter la hauteur de mousse initiale d'une solution aqueuse dudit mélange de tensioactifs d'au moins 5 mm par rapport à un témoin du mélange de tensioactifs sans polygalactomannane avec mise en oeuvre du test de mousse selon Ross-Miles.

4. Composition tensioactive moussable selon la revendication 1 dans laquelle ledit polygalactomannane est une gomme Guar ou un de ses dérivés.

5. Composition tensioactive moussable selon la revendication 2 dans laquelle ledit polygalactomannane est une gomme Guar ou un de ses dérivés.

6. Composition tensioactive moussable selon la revendication 3 dans laquelle ledit polygalactomannane est une gomme Guar ou un de ses dérivés.

7. Composition tensioactive moussable selon la revendication 1 dans laquelle ledit tensioactif est un oxyde d'alkylamine.

8. Composition tensioactive moussable selon la revendication 3 dans laquelle l'un desdits tensioactifs est un tensioactif anionique à base d'alkyl sulfate.

9. Composition tensioactive moussable selon la revendication 3 dans laquelle l'un desdits tensioactifs est une bétaïne à base de tensioactif zwitterionique.

10. Composition tensioactive moussable selon la revendication 3 dans laquelle l'un desdits tensioactifs est un tensioactif à base d'amphoglacinate.

11. Composition tensioactive moussable selon la revendication 3 dans laquelle l'un desdits tensioactifs est un tensioactif cationique à base d'oxyde d'alkylamine.

12. Composition tensioactive moussable selon la revendication 3 dans laquelle l'un desdits tensioactifs est un tensioactif anionique à base d'alkyléther sulfate.

13. Composition tensioactive moussable selon la revendication 3 dans laquelle ledit mélange de tensioactifs est mis en oeuvre en une quantité comprise entre environ 0,01 % et environ 20 % de la mousse finale.

14. Composition tensioactive moussable selon la revendication 3 dans laquelle ledit tensioactif est un mélange de tensioactifs zwitterioniques et anioniques.

15. Composition tensioactive moussable selon la revendication 3 dans laquelle ledit tensioactif est un mélange de tensioactifs à base d'oxyde d'amine et de lauryl sulfate de sodium.

16. Composition tensioactive moussable selon la revendication 3 sous forme concentrée dans laquelle ledit mélange de tensioactifs est mis en oeuvre en une quantité comprise entre environ 0,01 % et environ 20 % de la composition finale et le polygalactomannane est compris entre environ 0,0005 % et environ 50 %.

17. Composition tensioactive moussable selon la revendication 3 dans laquelle le polygalactomannane à base non aqueuse est mis en oeuvre en une quantité comprise entre environ 0,005 % et environ 50 % par rapport au poids des tensioactifs.

18. Composition tensioactive moussable selon la revendication 3 dans laquelle ledit polygalactomannane est âgé de moins de 24 heures après hydratation et avant transformation de la composition en mousse.

19. Composition tensioactive moussable selon la revendication 3 dans laquelle ledit polygalactomannane est âgé de moins de 3 heures après hydratation et avant transformation de la composition en mousse.

**20.** Composition tensioactive moussable selon la revendication 3 dans laquelle ledit polygalactomannane est âgé de moins d'1 heure après hydratation et avant transformation de la composition en mousse.

**21.** Composition tensioactive moussable caractérisée par une augmentation de la hauteur de mousse, contenant comme tensioactif :

a) un tensioactif ou un mélange de deux ou plusieurs tensioactifs non ioniques, anioniques, cationiques ou zwitterioniques en une quantité totale suffisante pour produire une mousse dans l'eau et

b) un polygalactomannane, y compris des mélanges, en une quantité suffisante pour augmenter la hauteur de mousse initiale d'une solution aqueuse dudit mélange de tensioactifs d'au moins 5 mm par rapport à un témoin du mélange de tensioactifs sans polygalactomannane avec mise en oeuvre du test de mousse selon Ross-Miles, dans laquelle ledit polygalactomannane est âgé de moins de 24 heures après hydratation et avant transformation de la composition en mousse.

**22.** Composition tensioactive moussable selon la revendication 21 dans laquelle ledit polygalactomannane est âgé de moins de 3 heures après hydration et avant transformation de la composition en mousse.

**23.** Composition tensioactive moussable selon la revendication 21 dans laquelle ledit polygalactomannane est âgé de moins d'1 heure après hydratation et avant transformation de la composition en mousse.

FIG. 1

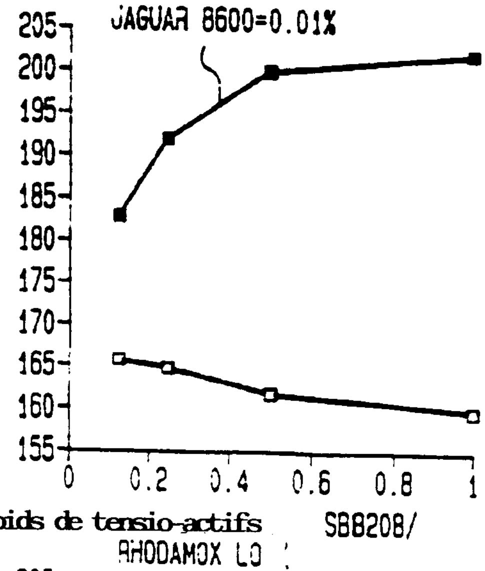


FIG. 2

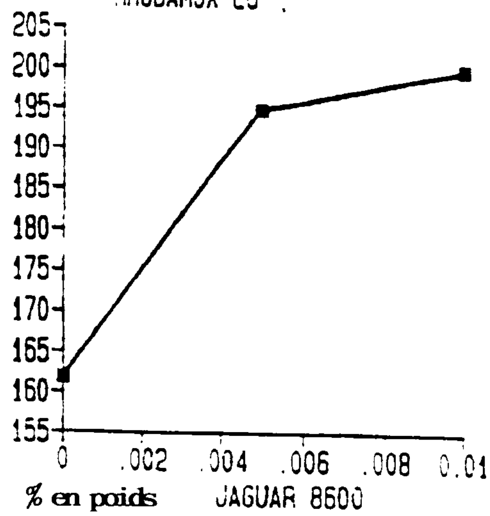


FIG. 3

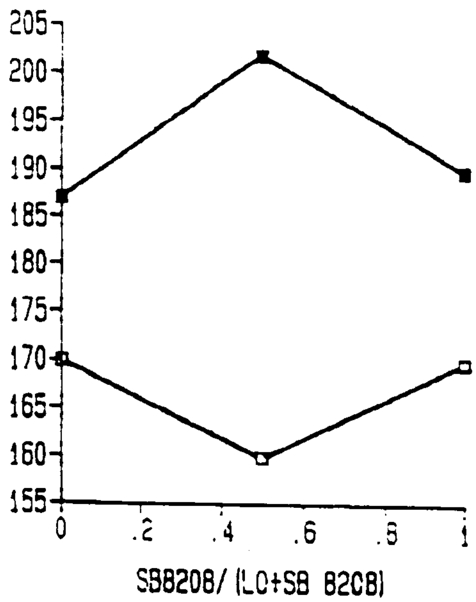

*FIG. 4*

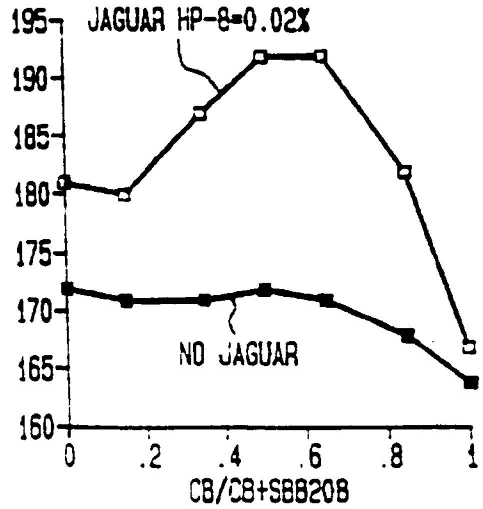


*FIG. 5*

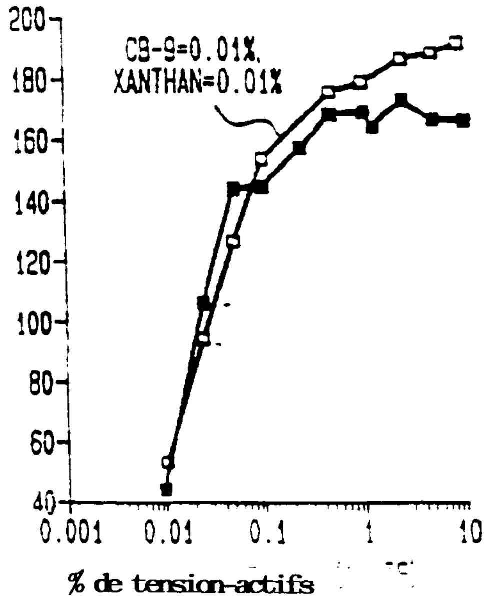


*FIG. 6*